Europäisches Patentamt

European Patent Office

Office européen des brevets

(11) Publication number: **0 226 903**
**B1**

(12) **EUROPEAN PATENT SPECIFICATION**

(45) Date of publication of patent specification: 26.09.90

(21) Application number: 86116854.0

(22) Date of filing: 04.12.86

(51) Int. Cl.⁵: **G 01 N 33/569,**
G 01 N 33/543,
G 01 N 33/546

(54) Immunoassay for antibodies to HTLV-III.

(30) Priority: 20.12.85 US 811240

(43) Date of publication of application:
01.07.87 Bulletin 87/27

(45) Publication of the grant of the patent:
26.09.90 Bulletin 90/39

(84) Designated Contracting States:
BE DE FR IT

(56) References cited:
EP-A-0 181 150
EP-A-0 187 041
EP-A-0 199 438
WO-A-86/06099

(73) Proprietor: ABBOTT LABORATORIES
14th Street and Sheridan Road North St
North Chicago Illinois 60064 (US)

(72) Inventor: Dawson, George L.
15670 Sprucewood Lane
Libertyville Illinois 60048 (US)

(74) Representative: Modiano, Guido et al
MODIANO, JOSIF, PISANTY & STAUB Modiano
& Associati Via Meravigli, 16
I-20123 Milano (IT)

Courier Press, Leamington Spa, England.

**Description**

Background of the Invention

Since human T-cell lymphotropic virus-III (HTLV-III) has been determined to be the probable causative agent of acquired immune deficiency syndrome (AIDS), several diagnostic immunoassays for antibodies to HTLV-III (anti-HTLV-III) have emerged. In U.S. Patent No. 4,520,113, three assays for anti-HTLV-III are described. These assays are a strip radioimmunoassay based on the Western Blot technique, an enzyme-linked immunosorbent assay (ELISA) and an indirect immunofluorescence assay.

The problem with these previous assay methods is that they result in a small percentage of false positives due to non-specific reactions caused by techniques or reagents. One example of such a non-specific reaction is the reaction between patients' samples and H-9 cellular protein materials in which the HTLV-III virus has been propagated for use as a reagent in the above-mentioned methods.

Since the report of a positive anti-HTLV-III assay can be devastating to a patient, all reactive samples should be confirmed as true positives by at least two assay procedures. Presently, test samples are most commonly screened by an ELISA method. Repeatedly reactive specimens are then confirmed by the Western Blot procedure.

The Western Bolt procedure generally described by Towbin et al., *Proc. Natl. Acad. Sci., 76:* 4350 (1979), requires sodium dodecylsulfate (SDS)-polyacrylamide gel electrophoresis to separate HTLV-III viral proteins based on their molecular weight. The separated HTLV-III proteins are then transferred to a nitrocellulose sheet by a second electrophoretic procedure. The nitrocellulose sheet is cut into strips and reacted with the test sample and then with a second antibody such as a radio-labeled goat anti-human IgG.

The disadvantages of the Western Blot procedure for detecting anti-HTLV-III are that it requires subjective interpretation, it is both complex and time-consuming to perform, and it is difficult to control variations in the composition of the HTLV-III viral proteins and performance of the test. Therefore, a need exists for both a more specific immunoassay procedure for anti-HTLV-III and a simple second method for confirming repeatedly reactive samples.

Summary of the Invention

The invention is a competitive immunoassay method for detection of antibody to HTLV-III in a biological sample comprising at least two detection systems. One detection system comprises:

a) coating a solid support with recombinant p24 protein expressed in *E. coli*;

b) contacting the p24-coated solid support with the biological sample and anti-HTLV-III-p24 conjugated to a label; and

c) detecting the label to determine the presence of anti-HTLV-III-p24 in the sample.

The other detection system comprises:

a) coating a solid support with recombinant p41 protein expressed in *E. coli*;

b) contacting the p41-coated solid support with the biological sample and anti-HTLV-III-p41 conjugated to a label; and

c) detecting the label to determine the presence of anti-HTLV-III-p41 in the sample.

The detection of antibody to HTLV-III-p24 in the one detection system or antibody to HTLV-III-p41 in the other detection system, or both, indicates the presence of anti-HTLV-III in the sample.

In a preferred embodiment, the solid support of one detection system is first coated with anti-HTLV-III-p24, and the solid support of the other detection system is first coated with anti-HTLV-III-p41 in order to enhance the coating of recombinant p24 and p41 proteins respectively on the solid supports.

Biological samples which are easily tested by the method of the present invention include human and animal body fluids. Solid supports which can be used in the immunoassay of the invention include wells of reaction trays, test tubes, beads, strips or other solid supports which are well known to those skilled in the art. Labels which can be used in the above-described assay include enzymes, radioisotopes and fluorescent labels.

Both polyclonal and monoclonal antibodies are useful as reagents in the present invention. Also, IgG and IgM antibodies may be used. In other preferred embodiment of the present invention, both detection systems of the invention are located on the same solid support.

Detailed Description of the Invention

The following examples are illustrative of the immunoassay of the present invention.

Example I

This example demonstrates an immunoassay for anti-HTLV-III in a human biological sample consisting of two solid phase detection systems. The first solid phase system comprises the following procedure:

1. A 6.35 mm (¼ inch) polystyrene bead is first coated with anti-HTLV-III-p24 and then with the recombinant core structural protein of HTLV-III primarily encompassing the p24 region. The p24-coated bead is then contacted with 50 µl serum, plasma, or other biological sample and 200 µl anti-HTLV-III-p24 conjugated to horseradish peroxidase (HRPO) for 1 to 24 hours and preferably 16 to 22 hours at a temperature range of 15°C to 45°C. The bead is then washed three times with 5.0 ml distilled water to remove any unbound sample or labeled antibody.

2. The washed bead is contacted with 300 µl of o-phenylenediamine-hydrogen peroxide solution which forms a yellow-colored product in the presence of horseradish peroxidase, and after incubating for approximately 30 minutes at room temperature, 1 ml of 0.5 m (1 N) $H_2SO_4$ is added.

3. Absorbance is read at 492 nm using a standard spectophotometer.

The second solid phase system comprises the following procedure:

1. A 6.35 mm ($\frac{1}{4}$ inch) polystyrene bead is first coated with anti-HTLV-III-p41 and then with the recombinant envelope protein of HTLV-III primarily encompassing the p41 region. The p41-coated bead is then contacted with 50 µl serum, plasma or other biological fluid and 200 µl anti-HTLV-III-p41 conjugated to horseradish peroxidase (HRPO) for 1 to 24 hours and preferably 16 to 22 hours at a temperature range of 20°C to 45°C. The bead is then washed three times with 5 ml distilled water to remove any unbound sample.

2. The washed bead is contacted with 300 µl of o-phenylenediamine-hydrogen peroxide solution which forms a yellow-colored product in the presence of horseradish peroxidase, and after incubating for approximately 30 minutes at room temperature, 1 ml of 0.5 m (1 N) $H_2SO_4$ is added.

3. Absorbance is read at 492 nm using a standard spectrophotometer.

In each detection system, the antibody of interest (anti-HTLV-III-p24 and anti-HTLV-III-p41) competes with the corresponding labeled antibody (anti-HTLV-III-p24-HRPO and anti-HTLV-III-p41-HRPO) for a number of binding sites on the coated bead. Therefore, the intensity of color developed in each detection system is inversely related to the amount of anti-HTLV-III-p41 and anti-HTLV-III-p24 in the sample. A positive reaction in either detection system or both indicates a positive test for anti-HTLV-III.

## Example II

This example demonstrates the specificity of the present invention. Serum samples from patients with AIDS, patients with lymphadenopathy syndrome (LAS), a condition which often precedes the development of full-blown AIDS and normal subjects were tested with the immunoassay described in Example I (Assay A), with a commercially available enzyme immunoassay for anti-HTLV-III, Abbott Laboratories, North Chicago, Illinois (Assay B) and by the Western Blot procedure (Assay C). All 91 of the AIDS patients' samples were positive by the Assay B procedure; 90 of 91 samples were positive by the Assay C procedure; and all samples were positive utilizing the Assay A procedure of the present invention (Table 1).

In the first detection system of the invention (which detects antibodies to HTLV-III-p24) 10 of 41 samples from individuals with AIDS were positive, while all 91 samples were positive utilizing the second detection system of the invention (which detects antibodies to HTLV-III-p41). Since a positive reaction in either detection system indicates a positive result, all samples were defined as positive by the inventive assay procedure.

All 62 samples from LAS patients were positive utilizing Assays A, B and C. None of the 375 samples from normal subjects had detectable antibody by Assays A, B and C. Thus, utilizing the assay of the present invention, all sera from AIDS patients and patients with LAS were detected as positive, while none of the sera from normal subjects were detected as positive.

## Example III

Samples of serum or plasma from blood donors which were repeatedly reactive in a commercially available enzyme immunoassay for anti-HTLV-III (Abbott Laboratories, North Chicago, Illinois), were tested with the present invention and by the Western Blot procedure (Table 2). Among these repeatedly reactive samples, 232 samples were negative by Western Blot and by the double detection system assay of the present invention. One sample which had an undeterminable reaction by the Western Blot procedure, was positive in the assay of the present invention, being reactive in both detection systems. Of the 118 samples confirmed positive by the Western Blot procedure, all 118 were also positive utilizing the present invention.

TABLE 1

Positive/Total

| Samples | Assay A | | Assay B | Assay C |
| | Anti-p24 | Anti-p41 | | |
|---|---|---|---|---|
| Normal Subjects | 0/375 | 0/375 | 0/375 | 0/375 |
| AIDS | 10/41 | 91/91 | 91/91 | 90/91 |
| LAS | 10/14 | 62/62 | 62/62 | 62/62 |

3

TABLE 2

Comparison of
Western Blot Procedure vs. Double Detection System Assay

| Samples (repeatedly reactive n = 351) | Positive in Either or Both Detection Systems | Positive in System 1 (anti-p24) | Positive in System 2 (anti-p41) |
|---|---|---|---|
| Western Blot negative (n = 232) | 0/232 | 0/232 | 0/232 |
| Western Blot positive (n = 118) | 118/118 | 102/118 | 115/118 |
| Western Blot undeter- minable (n = 1) | 1/1 | 1/1 | 1/1 |

The identification of biological samples which are positive for anti-HTLV-III is important in the screening and protection of human blood supplies. This assay enables a quick screening procedure as well as a second confirmatory assay for detecting true positive samples for anti-HTLV-III.

**Claims**

1. A method for detecting antibody to HTLV-III in a biological sample, comprising at least two detection systems, one detection system comprising:
   a. coating a solid support with recombinant p24 protein expressed in *E. coli*;
   b. contacting the p24-coated solid support with a biological sample and anti-HTLV-III-p24 conjugated to a label; and
   c. detecting the label to determine the presence of anti-HTLV-III-p24 in the sample;
   and the other detection system comprising:
   d. coating a solid support with recombinant p41 protein expressed in *E. coli*;
   e. contacting the p41-coated solid support with the biological sample and anti-HTLV-III-p41 conjugated to a label; and
   f. detecting the label to determine the presence of anti-HTLV-III-p41 in the sample;
   wherein the presence of anti-HTLV-III in either or both detection systems indicates the presence of anti-HTLV-III in the sample.

2. The method of Claim 1 wherein the solid support of step a. is first coated with anti-HTLV-III-p24 and then with the recombinant p24 protein; and the solid support of step d. is first coated with anti-HTLV-III-p41 and then with the recombinant p41 protein.

3. The method of Claim 1 wherein the labels of the two detection systems are enzymes, radioisotopes or fluorescent labels.

4. The method of Claim 1 wherein both detection systems are located on one solid support.

5. A method for detecting antibody to HTLV-III in a biological sample comprising at least two detection systems, the first detection system comprising:
   a. first coating a polystyrene bead with anti-HTLV-III-p24 and then with recombinant p24 protein expressed in *E. coli*;
   b. contacting the bead with a biological sample and anti-HTLV-III-p24 conjugated to horseradish peroxidase;
   c. washing the bead;
   d. contacting the washed bead with o-phenylenediamine-hydrogen peroxide solution to form a yellow-colored product; and
   e. detecting the absorbance of the yellow-colored product to determine the presence of anti-HTLV-III-p24 in the sample;
   and the second detection system comprising:
   f. first coating a polystyrene bead with anti-HTLV-III-p41 and then with recombinant p41 protein expressed in *E. coli*;
   g. contacting the bead with a biological sample and anti-HTLV-III-p41 conjugated to horseradish peroxidase;
   h. washing the bead;

4

EP 0 226 903 B1

i. contacting the washed bead with o-phenylenediamine-hydrogen peroxide solution to form a yellow-colored product; and

j. detecting the absorbance of the yellow-colored product to determine the presence of anti-HTLV-III-p41 in the sample.

6. The method of Claim 5 wherein the first and second detection systems are located on the same bead.

7. An immunoassay for detecting antibody to HTLV-III in a biological sample comprising at least two detection systems, the first detection system comprising:

a. a solid support coated with recombinant p24 protein expressed in *E. coli*; and

b. anti-HTLV-III-p24 conjugated to a label;

and the second detection system comprising:

c. a solid support coated with recombinant p41 protein expressed in *E. coli*; and

d. anti-HTLV-III-p41 conjugated to a label.

8. The immunoassay of Claim 7 wherein the solid support of the first detection system is first coated with anti-HTLV-III-p24 and then coated with the recombinant p24 protein, and the solid support of the second detection system is first coated with anti-HTLV-III-p41 and then coated with the recombinant p41 protein.

9. The immunoassay of Claim 7 wherein the labels of the first and second detection systems are enzymes, radioisotopes or fluorescent labels.

10. The immunoassay of Claim 7 wherein the first and second detection systems are located on the same solid support.


**Patentansprüche**

1. Ein Verfahren zum Nachweis eines Antiköropers von HTLV-III in einer biologischen Probe, enthaltend zumindest zwei Nachweissysteme, wovon das eine Nachweissystem enthält:

a. Beschichten eines festen Trägers mit rekombinantem p24 Protein exprimiert in *E. coli*,

b. Kontaktieren des p24-beschichteten festen Trägers mit einer biologischen Probe und Anti-HTLV-III-p24 konjugiert an einen Marker und

c. Nachweisen des Markers, um die Anwesenheit von Anti-HTLV-III-p24 in der Probe zu bestimmen, und worin das andere Nachweissystem enthält:

d. Beschichten eines festen Trägers mit rekombinantem p41 Protein exprimiert in *E. coli*,

e. Kontaktieren des p41-beschichteten festen Trägers mit einer biologischen Probe und Anti-HTLV-III-p41 konjugiert an einen Marker, und

f. Nachweisen des Markers, um die Anwesenheit von Anti-HTLV-III-p41 in der Probe zu bestimmen, worin die Anwesenheit von Anti-HTLV-III in einer oder beiden Nachweissystemen die Anwesenheit von Anti-HTLV-III in der Probe anzeigt.

2. Das Verfahren nach Anspruch 1, worin der feste Träger des Schritts a. zuerst beschichtet wird mit Anti-HTLV-III-p24 und dann mit dem rekombinanten p24 Protein und worin der feste Träger des Schritts d. zuerst beschichtet wird mit Anti-HTLV-III-p41 und dann mit dem rekombinanten p41 Protein.

3. Das Verfahren nach Anspruch 1, worin die Marker der zwei Nachweissysteme Enzyme, Radioisotope oder Fluoreszenzmarker sind.

4. Das Verfahren nach Anspruch 1, worin beide Nachweissysteme auf einem festen Träger lokalisiert sind.

5. Ein Verfahren zum Nachweis eines Antikörpers von HTLV-III in einer biologischen Probe, enthaltend zumindest zwei Nachweissysteme, wovon das erste Nachweissystem enthält:

a. zuerst Beschichten einer Polystyrolperle mit Anti-HTLV-III-p24 und dann mit rekombinantem p24 Protein, exprimiert in *E. coli*,

b. Kontaktieren der Perle mit einer biologischen Probe und Anti-HTLV-III-p24 konjugiert an Meerrettichbperoxidase,

c. Waschen der Perle,

d. Kontaktieren der gewaschenen Perle mit einer o-Phenylendiamin-Wasserstoffperoxidlösung unter Bildung eines gelbgefärbten Produktes und

e. Nachweisen der Absorption des gelbgefärbten Produktes, um die Anwesenheit von Anti-HTLV-III-p24 in der Probe zu bestimmen, und wovon das zweite Nachweissystem enthält:

f. zuerst Beschichten einer Polystyrolperle mit Anti-HTLV-III-p41 und dann mit rekombinantem p41 Protein, exprimiert in *E. coli*,

g. Kontaktieren der Perle mit einer biologischen Probe und Anti-HTLV-III-p41 konjugiert an Meerrettichperoxidase,

h. Waschen der Perle,

i. Kontaktieren der gewaschenen Perle mit einer o-Phenylendiamin-Wasserstoffperoxidlösung unter Bildung eines gelbgefärbten Produktes und

j. Nachweisen der Absorption des gelbgefärbten Produktes, um die Anwesenheit von Anti-HTLV-III-p41 in der Probe zu bestimmen.

5

6. Das Verfahren nach Anspruch 5, worin das erste und zweite Nachweissystem auf derselben Perle lokalisiert sind.

7. Ein Immunoassay zum Hachweis eines Antiköropers von HTLV-III in einer biologischen Probe, enthaltend zumindest zwei Nachweissysteme, wovon das erste Nachweissystem enthält.

a. einen festen Träger beschichtet mit rekombinantem p24-Protein exprimiert in *E. coli*, und

b. Anti-HTLV-III-p24 konjugiert an einen Marker, un wovon das zweite Nachweissystem enthält:

c. einen festen Träger beschichtet mit rekombinantem p41-Protein exprimiert in *E. coli*, und

d. Anti-HTLV-III-p41 konjugiert an einen Marker.

8. Der Immunoassay nach Anspruch 7, worin der feste Träger des ersten Nachweissystems zuerst mit Anti-HTLV-III-p24 beschichtet wird und dann mit dem rekombinanten p24 Protein beschichtet wird und worin der feste Träger des zweiten Nachweissystems zuerst mit Anti-HTLV-III-p41 beschichtet wird und dann mit dem rekombinanten p41 Protein beschichtet wird.

9. Der Immunosassay nach Anspruch 7, worin die Marker des ersten und zweiten Nachweissystems Enzyme, Radioisotope und Fluoreszenzmarker sind.

10. Der Immunoassay nach Anspruch 7, worin das erste und zweite Nachweissystem auf demselben festen Träger lokalisiert sind.

**Revendications**

1. Un procédé pour détecter l'anticorps du HTLV-III dans un échantillon biologique comprenant au moins deux systèmes de détection, l'un des systèmes de détection comprenant:

a. le revêtement d'un support solide avec la protéine p24 recombinante exprimée dans le *E. coli*;

b. la mise en contact du support solide revêtu de p24 avec un échantillon biologique et l'anti-HTLV-III-p24 conjugué à un marqueur; et

c. la détection du marqueur pour déterminer la présence de l'anti-HTLV-III-p24 dans l'échantillon; et l'autre système de détection comprenant:

d. le revêtement d'un support solide avec la protéine p41 recombinante exprimée dans le *E. coli*;

e. la mise en contact du support solide revêtu de p41 avec un échantillon biologique et l'anti-HTLV-III-p41 conjugué à un marqueur; et

f. la détection du marqueur pour déterminer la présence de l'anti-HTLV-III-p41 dans l'échantillon; la présence de l'anti-HTLV-III dans l'un ou l'autre système de détection ou dans les deux indiquant la présence de l'anti-HTLV-III dans l'échantillon.

2. Le procédé selon la revendication 1, selon lequel le support solide de l'étape a. est tout d'abord revêtu avec l'anti-HTLV-III-p24, puis avec la protéine p24 recombinante; et le support solide de l'étape d. est tout d'abord revêtu avec l'anti-HTLV-III-p41 et ensuite avec la protéine p41 recombinante.

3. Le procédé selon la revendication 1, selon lequel les marqueurs des deux systèmes de détection sont des enzymes, des radioisotopes ou des marqueurs fluorescents.

4. Le procédé selon la revendication 1, selon lequel les deux systèmes de détection sont localisés sur un support solide.

5. Un procédé pour détecter l'anticorpos du HTLV-III dans un échantillon biologique comprenant au moins deux systèmes de détection, le premier système de détection comprenant:

a. en premier lieu le revêtement d'une perle de polystyrène avec l'anti-HTLV-III-p24, puis avec la protéine p24 recombinante exprimée dans le *E. coli*;

b. la mise en contact de la perle avec un échantillon biologique et l'anti-HTLV-III-p24 conjugué à la peroxydase de raifort;

c. le lavage de la perle;

d. la mise en contact de la perle lavée avec un solution de o-phénylènediamine-peroxyde d'hydrogène pour former un produit coloré en jaune; et

e. la détection de l'absorbance du produit coloré en jaune pour déterminer la présence de l'anti-HTLV-III-p24 dans l'échantillon; et le second système de détection comprenant:

f. en premier lieu le revêtement d'une perle de polystyrène avec l'anti-HTLV-III-p41, puis avec la protéine p41 recombinante exprimée dans le *E. coli*;

g. la mise en contact de la perle avec un échantillon biologique et avec l'anti-HTLV-III-p41 conjugué à la peroxydase de raifort;

h. le lavage de la perle;

i. la mise en contact de la perle lavée avec un solution de o-phénylènediamine-peroxyde d'hydrogène pour former un produit coloré en jaune; et

j. la détection de l'absorbance du produit coloré en jaune pour déterminer la présence de l'anti-HTLV-III-p41 dans l'échantillon.

6. Le procédé selon la revendication 5, selon lequel les premier et second systèmes de détection sont localisés sur la même perle.

7. Un procédé de dosage immunologique pour détecter l'anticorps du HTLV-III dans un échantillon biologique comprenant au moins deux systèmes de détection, le premier système de détection comprenant:

6

**EP 0 226 903 B1**

a. un support solide revêtu de protéine p24 recombinante exprimée le *E. coli*; et

b. l'anti-HTLV-III-p24 conjugué à un marqueur;

et le second système de détection comprenant:

c. un support solide revêtu de protéine p41 recombinante exprimée dans le *E. coli*; et

d. l'anti-HTLV-III-p41 conjugué à un marqueur.

8. Le procédé de dosage immunologique selon la revendication 7, selon lequel le support solide du premier système de détection est en premier lieu revêtu avec l'anti-HTLV-III-p24, puis revêtu avec la protéine p24 recombinante, et le support solide du second système de détection est en premier lieu revêtu d'anti-HTLV-III-p41, puis revêtu avec la protéine p41 recombinante.

9. Le procédé de dosage immunologique selon la revendication 7, selon lequel les marqueurs des premier et second systèmes de détection sont des enzymes, des radioisotopes ou des marqueurs fluorescents.

10. Le procédé de dosage immunologique selon la revendication 7, selon lequel les premier et second systèmes de détection sont localisés sur le même support solide.

7